# EUROPEAN PATENT APPLICATION

(11) **EP 4 681 554 A1**
(43) Date of publication of application: **21.01.2026**
(21) Application number: 24189673.7
(22) Date of filing: 19.07.2024
(51) Int. Cl.: A23L 33/17, C12N 9/06, C12Q 1/26

(54) **DEGRADATION OF HARMFUL BIOGENIC AMINES IN FOOD WITH A NEW MICROBIAL ENZYME DERIVED FROM GLUTAMICIBACTER HALOPHYTOCOLA**

(71) Applicant: Universität Hohenheim, 70599 Stuttgart (DE)
(72) Inventor: Fischer, Lutz, 73274 Notzingen (DE); Kettner, Lucas, 70619 Stuttgart (DE); Lutz-Wahl, Sabine, 73230 Kirchheim unter Teck (DE)
(74) Representative: Müller-Boré & Partner Patentanwälte PartG mbB

(57) **Abstract**

The present invention relates to functional foods and dietary supplements comprising a specific diamine oxidase (DAO) enzyme derived from *Glutamicibacter halophytocola,* uses of said enzyme and respective methods for the production of biogenic amine-depleted products, uses of said enzyme as a biosensor for the analysis of one or more biogenic amine(s), as well as said enzyme for use in medicine, in particular for use in the prevention or treatment of a condition or disease that is associated with increased levels of biogenic amines.

## Description

The present invention relates to functional foods and dietary supplements comprising a specific diamine oxidase (DAO) enzyme derived from *Glutamicibacter halophytocola,* uses of said enzyme and respective methods for the production of biogenic amine-depleted products, uses of said enzyme as a biosensor for the analysis of one or more biogenic amine(s), as well as said enzyme for use in medicine, in particular for use in the prevention or treatment of a condition or disease that is associated with increased levels of biogenic amines.

Biogenic amines such as histamine and tyramine are mainly found in foods that undergo a fermentation process due to the presence of microorganisms, such as cheese, sausage, or wine, as well as in foods having increased levels of biogenic amines due to insufficient/inadequate cooling and/or conservation, and/or due to microbial contamination. The generation of L-histidine decarboxylase (EC 4.1.1.22) by these microorganisms leads to the formation of biogenic amines during the production and/or storage process in the food. The most important biogenic amines in this respect are histamine and tyramine which can cause toxicological effects in the human body.

Histamine acts as a hormone and neurotransmitter and regulates a number of important physiological functions in the human body. Therefore, the consumption of exogenous histamine can trigger a variety of allergy-like reactions in sensitive people (palpitations, increased blood pressure, itching, diarrhoea, flushing, etc.). The ingestion of large quantities of histamine can also lead to poisoning, which can for example result in hypotension or breathing difficulties. In special cases, however, even the consumption of moderate or small amounts of histamine can cause undesirable, allergy-like reactions. It is assumed in the scientific literature that around 1% of the global population is intolerant to histamine in food and therefore reacts sensitively to even small amounts.

In humans, the histamine present in food is normally metabolised by the enzyme diamine oxidase (DAO; EC 1.4.3.22) in the small intestine. This enzyme degrades histamine by oxidative deamination, resulting in the reaction products (imidazol-4-yl)acetaldehyde, hydrogen peroxide and ammonia. It is assumed that in people with histamine intolerance an imbalance between the histamine ingested with the food and the body's own DAO activity in the small intestine exists, so that histamine is not broken down sufficiently. In this context, DAO activity can be influenced by many factors, including individual genetics, alcohol consumption, medication, diet, among others.

There are currently no specific forms of treatment to combat the symptoms of intolerance against biogenic amines such as histamine. As a therapy, affected people are usually recommended a low histamine diet. Further, people who are affected can administer a dietary supplement containing porcine DAO that is supposed to support the human endogenous DAO in the small intestine. However, it has been shown that the activity required for a satisfactory histamine reduction is considerably larger than expected and that an alternative to the porcine DAO formulation used currently has to be found.

Specifically, DAO extracted from porcine kidney is commercially available in a dietary supplement and has already been investigated in clinical trials for its efficacy. However, it has recently been shown that this enzyme cannot be extracted and administered in sufficient quantities to achieve satisfactory histamine depletion. In particular, kinetic studies on porcine kidney DAO showed substrate inhibition by histamine concentrations greater than 56 mg/L (0.5 mM). The stability of free porcine DAO was tested in simulated intestinal fluid and showed a half-life of approximately 19 minutes. For the *in vitro* reduction of about 90% of histamine, a total of 50 nanokatal (nkat) free porcine DAO was required. This corresponded to the amount of enzyme isolated from about 100 g of pig kidney. The dietary supplement, containing a pig kidney extract did not show DAO activity. Instead, the histamine used in the experiment (0.75 mg) was apparently reduced by the adsorption to a capsule component by 18.9 ± 2.3% within 5 h. Although the capsule preparation retained its overall structure and shape for at least 90 min in simulated gastric fluid, the apparent histamine reduction was significantly reduced to 12.1 ± 2.3% (P ≤ 0.05). Thus, an alternative to porcine DAO is urgently needed to provide adequate supplementation for histamine-intolerant individuals.

In addition to DAO supplementation, DAO could also be used directly in foods or during fermentation to produce histamine-free or histamine-reduced foods. This general principle has already been successfully implemented industrially for the production of lactose-free foods. However, the commercially available DAO from pig kidneys cannot be provided in sufficient quantities for the production of histamine-free foods.

In particular, the use of DAO to break down histamine in food has already been demonstrated using an extremely high DAO activity of 42000 nkat/L in a tuna broth to degrade the histamine contained therein for the further production of a tuna paste. However, this enzyme activity used is so high that no economically viable industrial application is possible with DAO from pig kidney, since at least 80 kg of pig kidneys would be required to provide the enzyme activity for histamine degradation in one litre of tuna broth. In addition, a pH value of 7 would have to be present in the tuna broth for satisfactory histamine conversion.

An industrial application of the currently mainly used DAO from pig kidneys at acidic pH values (pH < 6) and low temperatures has not been described. In addition, this DAO can only be provided in low enzyme quantities and thus low specific enzyme activity by extraction and processing methods. However, a high specific enzyme activity is crucial for efficient use in the production of histamine-reduced foods. In addition, the DAO from pig kidney is not able to convert the important biogenic amine tyramine.

Sufficient DAO activities have already been provided by the discovery of a new microbial DAO, i.e., DAO-1 from *Yarrowia lipolytica.* This enzyme is particularly suitable for supplementation as a dietary supplement or drug due to its biochemical properties. However, this enzyme shows only very low activity at temperatures below 20 °C and at acidic pH values, e.g. at pH < 6. This limits in particular the use of this enzyme during food fermentation.

Accordingly, the technical problem underlying the present invention is the provision of improved means for the degradation of biogenic amines *in vitro* and *in vivo*, in particular with respect to food fermentation.

The solution to the above technical problem is achieved by the embodiments characterized in the claims.

In particular, in a first aspect, the present invention relates to a functional food, dietary supplement, or pharmaceutical composition, comprising an enzyme having diamine oxidase activity, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this context, the term "functional food" as used herein refers to any foodstuffs, e.g. any solid or liquid comestibles, e.g. foods and drinks, that are "functionalized" by the addition of the enzyme used in the present invention, i.e., that are modified to exhibit the additional effect of being able to reduce the levels of biogenic amines in a subject, preferably a human subject, upon ingestion of the functional food.

Further, the term "dietary supplement" as used herein refers to any manufactured product intended to supplement a subject's diet, preferably a human subject's diet, in the form of a pill, capsule, tablet, powder or liquid.

Particular foodstuffs, forms of dietary supplements, forms of pharmaceutical compositions, dosages, dosage regimens, and suitable formulations for the enzymes used in the present invention are not particularly limited and are known to the person skilled in the art and/or can be easily determined by the person skilled in the art.

The term "biogenic amines" as used herein relates to any amine compound that is a constituent of, secreted by or a metabolite of a plant, animal, fungus, or microorganism, provided that said amine can have an adverse effect on a subject. However, in preferred embodiments, the biogenic amine is selected from the group consisting of histamine, tyramine, putrescine, cadaverine, agmatine, spermidine, and tryptamine, preferably from the group consisting of histamine and tyramine. In specific embodiments, the biogenic amine is histamine.

In preferred embodiments, the above enzyme consists of
(i) the amino acid sequence of SEQ ID NO: 1; or
(ii) the amino acid sequence having at least 70% sequence identity to SEQ ID NO: 1 and having diamine oxidase activity.

In a specific embodiment, the above enzyme consists of the amino acid sequence of SEQ ID NO: 1.

As used herein, the term "enzyme having diamine oxidase activity" relates to an enzyme that catalyses the oxidative deamination of biogenic amines according to the general reaction

R-CH₂-NH₂ + H₂O + O₂ → R-CHO + NH₃ + H₂O₂.

The enzyme having diamine oxidase activity used in the present invention is either (i) the *Glutamicibacter halophytocola* diamine oxidase (DAO) (DAO-GH) having the amino acid sequence of SEQ ID NO: 1, the advantageous use of which in the degradation of biogenic amines has been discovered in the present invention, or (ii), in the case of an enzyme having at least 70% sequence identity to SEQ ID NO: 1 and having diamine oxidase activity, a respective DAO derived therefrom.

The term "enzyme comprising an amino acid sequence having at least 70% sequence identity to SEQ ID NO: 1 and having diamine oxidase activity" relates to polypeptides that can comprise any number of amino acid substitutions, additions, or deletions with respect to the amino acid sequence of SEQ ID NO: 1, e.g. 1, 2, 3, 4, 5, 6, 7, 8, 9, 10, or any number up to 195 (i.e., any integer n, wherein 1 ≤ n ≤ 195), amino acid substitutions, additions, or deletions with respect to the amino acid sequence of SEQ ID NO: 1, provided that the resulting polypeptides fulfil the requirement of having at least 70% sequence identity to SEQ ID NO: 1 and retain biological activity of a diamine oxidase. In this context, the term "retains the biological activity of a diamine oxidase" as used herein relates to polypeptides that have at least 5%, at least 10%, at least 15%, at least 20%, at least 25%, at least 30%, at least 35%, at least 40%, at least 45%, at least 50%, preferably at least 55%, at least 60%, at least 65%, at least 70%, at least 75%, at least 80%, at least 82%, at least 84%, at least 86%, at least 88%, at least 90%, at least 91 %, at least 92%, at least 93%, at least 94%, at least 95%, at least 96%, at least 97%, at least 97.5%, at least 98%, at least 98.5%, at least 99%, at least 99.5%, 100%, or more than 100% of the activity of the *G*. *halophytocola* DAO having the amino acid sequence of SEQ ID NO: 1, as determined in a standard diamine oxidase activity assay known in the art.

While the number of amino acid substitutions, additions, or deletions is generally only limited by the above proviso concerning the sequence identity, biological activity of the resulting polypeptide, and absolute number of amino acid substitutions, additions or deletions, it is preferable that the resulting polypeptide has at least 72%, at least 74%, at least 76%, at least 78%, at least 80%, at least 82%, at least 84%, at least 86%, at least 88%, at least 90%, at least 91 %, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1%, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

Means for determining the sequence identity of an amino acid sequence to a reference sequence are known in the art.

In a further aspect, the present invention relates to the use of an enzyme having diamine oxidase activity in the production of a biogenic amine-depleted product, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the functional food or dietary supplement of the present invention apply in an analogous manner. In particular, the enzyme as such and the biogenic amines are as defined above.

Means of using the enzyme used in the present invention in the production of a biogenic amine-depleted product are not particularly limited and are known in the art. Respective means include for example the step of contacting (i) a biogenic amine-containing product, and/or (ii) a biogenic amine-containing intermediate product of said product, with said enzyme under conditions and for a duration of time suitable to degrade a biogenic amine present in said product and/or in said intermediate product of said product. Respective conditions and or durations of time are not particularly limited and are known to the person skilled in the art and/or can be easily determined by the person skilled in the art. These include for example the incubation of the product or intermediate product thereof with the enzyme in an amount providing an enzyme activity of 0.1 to 10 nkat/ml or 0.1 to 10 nkat/mg, e.g. an enzyme activity of 0.1 nkat/mL or 0.1 nkat/mg, of the product or intermediate product thereof within at most 5 h at a temperature in a range of about 5 °C to about 20 °C, preferably in a range of about 8 °C to about 15 °C, e.g. at about 12 °C, and/or at an acidic pH, e.g. at a pH in the range of about pH 4.5 to about pH 6.9, preferably in a range of about pH 4.9 to about pH 6.4.

The term "biogenic amine-depleted product" as used herein refers to a product whose biogenic amine content has been reduced with respect to its original biogenic amine content. In specific embodiments, the biogenic amine content has been reduced by at least 50%, at least 55%, at least 60%, at least 65%, at least 70%, at least 75%, at least 80%, at least 85%, at least 90%, at least 95%, at least 95.5%, at least 96%, at least 96.5%, at least 97%, at least 97.2%, at least 97.4%, at least 97.5%, at least 97.6%, at least 97.7%, at least 97.8%, at least 97.9%, at least 98%, at least 98.1%, at least 98.2%, at least 98.3%, at least 98.4%, at least 98.5%, at least 98.6%, at least 98.7%, at least 98.8%, at least 98.9%, at least 99%, at least 99.1%, at least 99.2%, at least 99.3%, at least 99.4%, at least 99.5%, at least 99.6%, at least 99.7%, at least 99.8%, or at least 99.9%.

Products amenable to the above use according to the present invention are not particularly limited and include any products containing biogenic amines in which the reduction of biogenic amine content might be of interest. Preferably, the product is selected from the group consisting of foodstuffs and feedstuffs. In this context, the term "foodstuffs" as used herein refers to any solid or liquid comestibles, e.g. foods and drinks, that can be ingested by a human for nutritional and/or recreational purposes. Likewise, the term "foodstuffs" as used herein refers to any solid or liquid comestibles, e.g. feeds, that can be ingested by an animal for nutritional purposes. In specific embodiments, the above product is a foodstuff, selected from the group consisting of fermented foodstuffs, such as cheeses, sauerkraut, sausages, wine, chocolate, and yeast extracts, and fish and fish products, raw meats, and fresh milk.

In a related further aspect, the present invention relates to a method for the production of a biogenic amine-depleted product, comprising the step of contacting (i) a biogenic amine-containing product, and/or (ii) a biogenic amine-containing intermediate product of said product, with an enzyme having diamine oxidase activity under conditions and for a duration of time suitable to degrade a biogenic amine present in said product and/or in said intermediate product of said product, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the functional food or dietary supplement of the present invention and/or the use of the present invention apply in an analogous manner. In particular, the enzyme as such, the product, the term "biogenic amine-depleted product", and the biogenic amines are as defined above.

Respective conditions and or durations of time applicable in the methods of the present invention are not particularly limited and are known to the person skilled in the art and/or can be easily determined by the person skilled in the art. These include for example the incubation of the product or intermediate product thereof with the enzyme as defined above for the uses of the present invention.

In a further aspect, the present invention relates to a product obtained by the above method.

In this aspect, all relevant definitions and limitations indicated above for the functional food or dietary supplement of the present invention, the use of the present invention, and/or the method of the present invention apply in an analogous manner. In particular, the enzyme as such, the product, the term "biogenic amine-depleted product", and the biogenic amines are as defined above.

In a further aspect, the present invention relates to an enzyme having diamine oxidase activity for use in medicine, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the functional food or dietary supplement of the present invention, the use of the present invention, and/or the method of the present invention apply in an analogous manner. In particular, the enzyme as such is as defined above.

In specific embodiments, the above enzyme is for use in a method of preventing or treating a condition or disease that is associated with increased levels of biogenic amines in a subject, preferably a mammalian subject, more preferably a human subject.

In this context, the term "increased levels of biogenic amines" as used herein refers to increased levels of biogenic amines *in vivo*, e.g. in the small intestine, where biogenic amines first accumulate before passing into the bloodstream, or in the blood of the subject. Means for determining the level of biogenic amines in a subject are not particularly limited and are known in the art.

Increased *in vivo* levels of biogenic amines can result from pathological conditions in the body, e.g. dysregulated immune functions as in allergic reactions, or can result from the ingestion of biogenic amines by the subject, e.g. by consuming foodstuffs containing high levels of biogenic amines.

In specific embodiments, the condition or disease that is associated with increased levels of biogenic amines is selected from the group consisting of allergies, acute and chronic allergic diseases, allergic reactions, allergy-like reactions, itching (pruritus), diarrhoea, redness (erubescence), vomiting (emesis), acute and chronic biogenic amine poisoning, hypotonia, difficulty of breathing, biogenic amine intolerance, anaphylaxis, anaphylactic shock, acute and chronic urticaria, asthma, hay fever, allergic rhinitis, allergic conjunctivitis, headache, migraine, atopic dermatitis, mastocytosis, mast cell activation syndrome (MCAS), pre-eclampsia, hyperemesis gravidarum, pre-term labour, peptic ulcers, acid reflux, sepsis, fibromyalgia, chronic fatigue syndrome, and spondylitis.

In a related aspect, the present invention relates to a method of preventing or treating a condition or disease that is associated with increased levels of biogenic amines in a subject, comprising the step of administering an enzyme having diamine oxidase activity to the subject, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the enzyme for use of the present invention apply in an analogous manner. In particular, the enzyme as such, the condition or disease that is associated with increased levels of biogenic amines in a subject, the subject, and the biogenic amines are as defined above.

In this context, dosages, dosage regimens, administration modes and suitable formulations for the enzymes used in the present invention are not particularly limited and are known to the person skilled in the art and/or can be easily determined by the person skilled in the art.

In a further aspect, the present invention relates to the use of an enzyme having diamine oxidase activity in a functional food or in a dietary supplement, wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the functional food or dietary supplement of the present invention, apply in an analogous manner. In particular, the enzyme as such, the functional food, and the dietary supplement are as defined above.

In a final aspect, the present invention relates to the use of an enzyme having diamine oxidase activity as a biosensor for the analysis of one or more biogenic amine(s),
wherein said enzyme comprises
(i) the amino acid sequence of SEQ ID NO: 1; or
(ii) an amino acid sequence having at least 70% sequence identity to SEQ ID NO: 1 and having diamine oxidase activity.

In this aspect, all relevant definitions and limitations indicated above for the functional food or dietary supplement of the present invention, apply in an analogous manner. In particular, the enzyme as such, and the biogenic amines are as defined above.

With increasing awareness of the potential toxicity of biogenic amines, especially of histamine and tyramine, there is an increasing demand for biosensors for the quick and reliable detection in foods. Therefore, research was done to investigate different DAOs and techniques for the development of biogenic amine biosensors. However, there are disadvantages of the currently used DAOs, which are:
1) The substrate selectivity of DAOs currently used is often not sufficient for the development of a biosensor that detects both histamine and tyramine. If porcine DAO is used, tyramine cannot be detected and if a plant DAO is used (e.g. from *Lathyrus sativus*) the activity with histamine is far lower in comparison to other biogenic amines such as putrescine and cadaverine, which leads to a lower sensitivity.
2) If porcine or plant DAOs are used, other biogenic amines such as putrescine and cadaverine are also accepted and therefore detected. This biosensor would not be specific for the detection of the most toxic biogenic amines histamine and tyramine.

The DAO-GH from *Glutamicibacter halophytocola* of this present invention solves all the challenges listed above, since it shows 1) high affinity towards histamine and tyramine over a broad and food-relevant concentration range and 2) negligible activity with the biogenic amines putrescine and cadaverine. Therefore, the DAO-GH can be used as a sensitive and histamine/tyramine-specific biosensor.

As used herein, the term "comprising"/"comprises" expressly includes the terms "consisting essentially of"/"consists essentially of" and "consisting of"/"consists of", *i.e.*, all of said terms are interchangeable with each other herein.

Further, as used herein, the term "about" represents a modifier of ± 10% of the specified value, preferably ± 7.5%, ± 5%, ± 3%, ± 2%, or ± 1% of the specified value. Thus, by way of example, the term "about 10" includes the ranges 9 to 11, 9.25 to 10.75, 9.5 to 10.5, 9.7 to 10.3, 9.8 to 10.2, and 9.9 to 10.1.

In the present invention, in particular for applications with respect to the production of histamine-free or histamine-reduced foods, a new DAO was discovered in the bacterium *Glutamicibacter halophytocola* (DAO-GH), which has particularly interesting biochemical properties. The DAO-GH was produced recombinantly in *Komagataella phaffii.* For this purpose, the DAO-GH gene was integrated into the genome of *K. phaffii* (AOX1 locus). Gene expression was controlled by the methanol-inducible AOX1 promoter. In a bioreactor cultivation of the recombinant *K. phaffii,* an activity of at least 65,000 nkat/L_{bioreactor} was achieved. A specific enzyme activity of about 9 µkat/g protein was achieved by purification using polyethyleneimine and ammonium sulphate precipitation and hydrophobic interaction chromatography. With histamine as substrate, the DAO-GH still exhibits about 60 % of the maximum activity at pH 6. Even at pH 5, around 4 % of the DAO activity is still present. With tyramine as substrate, the DAO-GH exhibits around 100 % activity at pH 6. Surprisingly, at pH 4.5 and 5, the DAO-GH shows around 5 % and 4 % with tyramine, respectively. At a temperature of just about 5 °C, 14 % of the activity with histamine is present (compared to the maximum activity at 40 °C). With tyramine, around 12 % of the activity is present at 5°C (compared to the activity at 40 °C). Furthermore, DAO-GH has a high affinity for histamine (Km = 0.9 mM). In addition to histamine, the highly food-relevant biogenic amine tyramine is also efficiently converted with a very high affinity (Km = 0.009 ± 0.0002 mM). In bioconversion studies with the two biogenic amines (150 mg/L, pH 6.8, 37 °C, 5 h, 0.1 nkat/mL), almost complete conversion (approx. 95 %) was achieved in a simulated food matrix (5 g/L bovine serum albumin, 25 g/L sucrose) and with the addition of catalase (30 nkat/mL). The aforementioned biochemical characteristics (good producibility, sufficient activity at low pH and low temperature, high affinity towards the most relevant biogenic amines histamine and tyramine) are unique to DAO-GH and have not yet been found in this combination in any other microbial DAO.

A DAO-GH activity of 64,000 nkat can currently be produced in a bioreactor volume of only 1 litre. However, it can be assumed that much higher activity yields can be realised in the future by further improving the expression system and the cultivation strategy. Thus, sufficient enzyme activities can be provided for industrial application.

The newly discovered enzyme could be used to produce safe, histamine- and/or tyramine-reduced, or histamine- and/or tyramine-free foods.

The aim of the present invention was to provide a robust microbial diamine oxidase (DAO) that is suitable for the safe production of histamine- and/or tyramine-reduced/free foods. For this purpose, it is necessary that the DAO has a stable, high relative enzyme activity (≥ 3 %) in a pH (pH 4.5 - 6.9) and temperature range (5 - 20 °C) relevant for food fermentations. Such an enzyme did not exist before. However, the newly discovered DAO-GH is able to efficiently break down the amounts of histamine and tyramine normally found in foodstuffs under the above-mentioned practical conditions. Furthermore, the microbial DAO-GH can be produced in large quantities in the bioreactor and can subsequently be purified to a high specific enzyme activity at low cost. This should enable the enzyme to be used efficiently in foods for the degradation of the most important biogenic amines, histamine, and tyramine.

The present diamine oxidase from *Glutamicibacter halophytocola* (DAO-GH) shows a relative activity of > 4 - 90 % in the acidic pH range relevant for food fermentations (based on the pH optimum of 7 with histamine and 6.8 with tyramine). It also shows a relative activity of > 12 - 35 % (in comparison to the activity at 40 °C, both for histamine and tyramine) in the temperature range relevant for food fermentations (5 - 20 °C). The DAO-GH also has a high substrate affinity for histamine with a Km value of 0.9 mM. In addition to histamine, it also converts tyramine and shows a very high affinity (Km = 0.009 ± 0.0002 mM) to the same. When converting histamine (1.35 mM corresponds approx. 150 mg/L) in a simulated food matrix (25 g/L sucrose, 5 g/L bovine serum albumin and 30 nkat/L catalase) at 37 °C and pH 6.8, DAO-GH (0.1 nkat/mL) was able to degrade approx. 95 % of histamine after 5 hours. When tyramine was converted under the same conditions, approx. 95 % tyramine was also degraded after 5 h.

The microbial diamine oxidase from *Glutamicibacter halophytocola* (DAO-GH) of the present invention is a new, previously unknown enzyme. This enzyme has surprising, previously unknown properties for use in the food industry for the targeted degradation of histamine and tyramine directly during food production. It has a high affinity for the most food-relevant biogenic amines (histamine and tyramine). The biochemical characteristics mentioned above (economic producibility, sufficient activity at low pH and low temperature, high affinity towards histamine and tyramine) are unique to DAO-GH and have not yet been found in this combination in any other DAO. In addition, the cost-effective and efficient production of DAO-GH in *K. phaffii* makes it preferably a suitable enzyme for the degradation of biogenic amines in food, but also for use as a dietary supplement/medication.

### The figures show:

Figure 1:
   Cassette plasmid for the integration of the DAO-GH gene into the genome of *Komagataella phaffii* ATCC 76273. Pmel linearization of the cassette plasmid prior to transformation in *K. phaffii.*
Figure 2:
   *Glutamicibacter halophytocola* grown on minimal media agar plates containing 0.4 and 4 g/L histamine. *G. halophytocola* (cultivated in S1-media) was first diluted to an OD₆₀₀ of 1 in saline (0.9 % (w/v)) before it was serially diluted (F 10-1000) in saline and plated on the minimal media agar plates.
Figure 3:
   Investigation of intracellular, recombinant DAO-GH production in *K. phaffii* using the AOX1 promoter. Cultivation was done in deep-well plates in 500 µL working volume at 30 °C using BMGY/BMMY medium. DAO activity was determined after 48 h of cultivation.
Figure 4:
   Fed-batch bioreactor cultivation of the recombinant *K. phaffii* (pAOX1) for the production of DAO-GH. BSM medium [carbon sources: glycerol (batch) and methanol (fed-batch)], 30 °C, pH 5.
Figure 5:
   Chromatogram of the HIC purification of DAO-GH. DAO-GH was eluted in a linear gradient by decreasing the binding buffer and thereby the ammonium sulfate concentration from 1.3 M to zero.
Figure 6:
   SDS-PAGE analysis of the purified DAO-GH (10 % (w/v) SDS-gel). Staining was done using Coomassie brilliant blue R-250. 5 µg of protein of purified DAO-GH was loaded on the gel. M = Precision Plus Protein ^{™} unstained protein standard 10 - 250 kDa.
Figure 7:
   Dependence of DAO-GH activity on the temperature with histamine as substrate. The DAO-GH activity was determined in potassium phosphate buffer (25 mM, pH 6.8). 100 % DAO activity = 6.7 ± 0.09 nkat/mg.
Figure 8:
   Dependence of DAO-GH activity on the temperature with tyramine as substrate. The DAO-GH activity was determined in potassium phosphate buffer (25 mM, pH 6.8). 100 % DAO activity = 12.5 ± 0.09 nkat/mg.
Figure 9:
   Dependence of DAO-GH activity on the pH-value and buffer (each at 50 mM) with histamine as substrate. The DAO-GH activity was determined at 37 °C. 100 % DAO activity = 7.0 ± 0.02 nkat/mg.
Figure 10:
   Dependence of DAO-GH activity on the pH-value and buffer (each at 50 mM) with tyramine as substrate. The DAO-GH activity was determined at 37 °C. 100 % DAO activity = 10.8 ± 0.03 nkat/mg.
Figure 11:
   Michaelis-Menten kinetics of the DAO-GH with histamine as substrate. The DAO-GH activity was determined in potassium phosphate buffer (25 mM, pH 6.8) at 37 °C.
Figure 12:
   Michaelis-Menten kinetics of the DAO-GH with tyramine as substrate. The DAO-GH activity was determined in potassium phosphate buffer (25 mM, pH 6.8) at 37 °C.

The present invention will be further illustrated by the following examples without being limited thereto.

### Examples

### Material and methods:

### Materials and reagents

Yeast extract was purchased from Acros Organics (New Jersey, USA). Tryptone (enzymatic digest from casein), sodium diethyldithiocarbamate and orthophosphoric acid (H₃PO₄) were obtained from Merck KGaA (Darmstadt, Germany). 1,4-piperazinediethanesulfonic acid (PIPES), MES, TRIS, sodium acetate, histamine dihydrochloride, tyramine dihydrochloride, sodium hydroxide (NaOH), monobasic potassium phosphate (KH₂PO₄), di-potassium hydrogen phosphate (K₂HPO₄), ammonium sulfate, hydrochloric acid (HCl), hydrogen peroxide (30 %), sodium chloride (NaCl), glucose, sorbitol and agar-agar were purchased from Carl Roth GmbH (Karlsruhe, Germany). Peptone (from casein, enzymatic digest) and polyethyleneimine solution (average Mₙ ~60,000 by GPC, average M_{w} ~750,000 by LS, 50 wt. % in H₂O) were purchased from Sigma-Aldrich. Zeocin was purchased from InvivoGen (San Diego, USA). Kanamycin sulfate, Bovine serum albumin (BSA; modified Cohn Fraction V, pH 5.2) and chloramphenicol were purchased from Serva electrophoresis GmbH (Heidelberg, Germany). TaKaRa Ex Taq^{®} DNA polymerase was purchased from Takara Bio Inc. (Kusatsu, Japan). The Precision Plus Protein^{™} unstained protein standard 10-250 kDa was purchased from Bio-Rad laboratories GmbH (Feldkirchen, Germany). (10-(carboxymethyl-aminocarbonyl)-3,7-bis(dimethylamino) phenothiazine sodium salt (DA-67) was purchased from Fujifilm Wako Chemicals U.S.A. Corp (Richmond, USA). Horseradish peroxidase (Grade I) was purchased from AppliChem GmbH (Darmstadt, Germany).

### Strains and media

The minimal media used for the preparation of screening agar plates contained 5 g/L glycerol and glucose, 4 or 0.4 g/L histamine, 2 g/L K₂HPO₄, 1 g/L KH₂PO₄, 4 mL/L trace element solution 1 (18.25 g/L citric acid, 10 g/L FeSO₄ × 7 H₂O, 1.25 g/L MnSO₄ × H₂O, 10 g/L CaCl₂ × 6 H₂O, 10 g/L MgCl₂; pH adjusted to 7 with 5 M NaOH) and 4 mL/L trace element solution 2 (2.5 g/L citric acid, 0.1 g/L NaMoO₄ × 2 H₂O, 0.1 g/L CoCl₂ × 6 H₂O, 0.1 g/L CuCl₂ × 2 H₂O, 6.25 g/L ZnSO₄ × 7 H₂O; pH adjusted to 4 with 5 M NaOH). All components were separately prepared as stock solutions and sterile filtered (except glucose and glycerol, which were sterilized at 121 °C and 1 bar overpressure). For the preparation of agar plates, 15 g/L Bacto^{™} Agar (Becton, Dickinson and Company, Franklin Lakes, USA) was used.

*Glutamicibacter halophytocola* was cultivated in a modified S1 complex media containing 15 g/L peptone, 3 g/L yeast extract, 6 g/L NaCl, 1 g/L glucose, 1 g/L glycerol and 1 mM histamine (if stated in the text) (pH adjusted to pH 7 with 5 M NaOH).

Plasmid construction and propagation was done in *Escherichia coli* XL-1, grown in lysogeny broth media with the appropriate antibiotic (30 µg/mL kanamycin or 40 µg/mL chloramphenicol). *Komagataella phaffii* ATCC 76273 (also known as CBS7435 or NRRL Y-11430) was purchased from the American Type Culture Collection and cultivated at 30 °C. *K. phaffii* transformants were selected on YPDS agar plates (10 g/L yeast extract, 20 g/L peptone, 20 g/L glucose, 1 *M* sorbitol, 15 g/L agar-agar) containing 100 µg/mL zeocin. The following media were used for cultivation of recombinant *K. phaffii* strains: YPD medium (10 g/L yeast extract, 20 g/L peptone, 20 g/L glucose), BMGY or BMMY medium (10 g/L yeast extract, 20 g/L peptone, 100 m *M* potassium phosphate buffer (pH 6), 13.4 g/L YNB, 0.4 mg/L biotin, 10 g/L glycerol or 5 mL/L methanol), BMG medium (100 m*M* potassium phosphate buffer (pH 5), 13.4 g/L YNB, 0.4 mg/L biotin, 10 g/L glycerol) and BSM medium (26.7 mL/L phosphoric acid (85 %), 0.93 g/L calcium sulfate, 18.2 g/L potassium sulfate, 14.9 g/L magnesium sulfate-7·H₂O, 4.13 g/L potassium hydroxide, 40 g/L glycerol at start of batch phase) containing 4.35 mL/L PTM₁ trace salts solution (Invitrogen^{™}).

### Activity-based screening for diamine oxidase producing microorganisms and identification

A soil sample was taken from under a chive bush (Stuttgart, Germany) and used to screen for diamine oxidase-producing microorganisms. Besides this soil sample, several other environmental samples were used for the screening. For the sake of simplicity, only the soil sample from the chive bush is described, investigated and discussed below.

250 mg soil were suspended in 1 mL saline (0.9 % (w/v) NaCl) and subsequently diluted 1,000-fold. 100 µL of the diluted soil sample were plated on the selective minimal media agar plate that contained histamine (4 g/L) as the sole nitrogen source. The agar plate was incubated at 30 °C for 48 h. Colonies of different morphologies were picked and streaked on a fresh minimal media agar plate for isolation and incubated at 30 °C for 48 h. Single colonies were picked and used to inoculate test tubes with 1.5 mL S1 complex media supplemented with glycerin and incubated for 24 h at 30 °C, 180 rpm. Cells were harvested by centrifugation (5,000 rpm, 5 min, 4 °C) and washed with saline. A cell suspension of each candidate, standardized to an optical density (OD₆₀₀) of 1, was prepared by dilution with saline. The cell suspension was serially diluted (10- to 1,000-fold) and 5 µL of each dilution was spotted on minimal media screening agar plates with 4 and 0.4 g/L histamine and incubated at 30 °C for 20 h.

Separately, the candidates were used to inoculate deep-well plate cultivations in 500 µL S1 media (with glycerin and the addition of 1 mM histamine). The deep well plate was covered with a Breathe Easier sealing membrane (Sigma-Aldrich, USA) and incubated for 20 h at 30 °C, 900 rpm. Cells were harvested by centrifugation (3,000 rpm, 10 min, 4 °C) and cell pellets were washed with 500 µL saline. After centrifugation (3,000 rpm, 10 min, 4 °C) the supernatant was discarded and 200 µL of PIPES buffer (pH 7.2) supplemented with lysozyme (1 mg/mL) were added to suspend the cells. After incubation (37 °C, 500 rpm, 20 min) the cell suspension was transferred to a microtiter plate containing 200 mg glass beads (0.5-0.7 mm diameter) in each well. Cells were disrupted at 30 Hz for 20 min in a TissueLyser II (Qiagen, Germany). After centrifugation (1,500 rpm, 10 min, 4 °C), the supernatant was directly used for DAO activity determination. For the identification, each candidate was cultivated in S1 complex media for 24 h at 30 °C, 180 rpm and subsequently genomic DNA was extracted and purified using the Thermo Scientific^{™} GeneJET genomic DNA purification Kit (Fisher Scientific, USA). Identification was done by 16s rDNA sequencing (Eurofins Genomics GmbH, Germany) and evaluation using the National Center for Biotechnology Information (NCBI) database.

The candidate with the best growth on the minimal media agar with the lowest histamine concentration (0.4 g/L) and the overall highest native DAO activity was cultivated in a shake flask (50 mL S1 media, supplemented with glycerin and histamine) to verify native DAO activity. The cultivation was done at 30 °C, 110 rpm for 25 h and the OD₆₀₀ was regularly measured over the course of the cultivation. Samples of 10 mL that were taken for DAO activity determinations were harvested by centrifugation (5,000 g, 10 min, 4 °C), washed with saline and again pelleted by centrifugation. Cells were disrupted in PIPES buffer (pH 7.2) supplemented with lysozyme (1 mg/mL) using glass beads (0.75 mm diameter) in a TissueLyser II (Qiagen, Germany). After centrifugation (6,000 rpm, 5 min, 4 °C), the supernatant was directly used for DAO activity determination.

The gene coding for the DAO in this candidate was identified *in silico* using the BLAST program (https://blast.ncbi.nlm.nih.gov) with the amino acid sequences of the DAOs from *Yarrowia lipolytica* (accession: Q6CGT2) and *Arthrobacter crystallopoietes* (accession: WP_074701741.1) as entries. This gene was amplified by PCR from genomic DNA of the candidate *G. halophytocola* using the primers 5'-ATGGAACACCTTCACCCAACGA-3' (SEQ ID NO: 4) and 5'-CTAGGCTCCGCAGTGTCCTTCGGTTTCAGG-3' (SEQ ID NO: 5).

### Construction and investigation of recombinant K. phaffii clones

The gene of the DAO-GH from *G. halophytocola* was codon-optimized for the production in *Komagataella phaffii* and synthesized by Invitrogen (Thermo Fisher Scientific, Waltham, Massachusetts, USA).

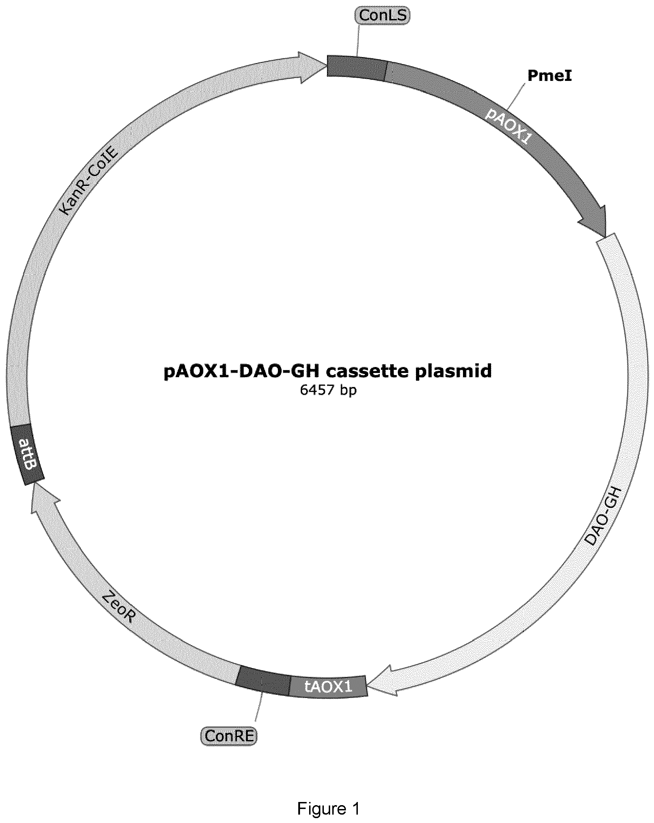

The construction of the cassette plasmid containing the DAO-GH expression cassette was done with the MoClo Yeast and MoClo *Pichia* Toolkit, as known in the art. As also known in the art, the DAO-GH gene was flanked by *Bsm*BI and Bsal restriction sites and specific overhangs of a type 3 part. Part plasmids used for the cassette plasmid assembly are shown in Table 1 below. The cassette plasmid map is shown in Fig. 1.

**Table 1: Part plasmids used for cassette plasmid construction.**

| **Plasmid** | **Type** | **Description/Parts** |
|---|---|---|
| pYTK002 | 1 | ConLS (assembly connector) |
| pPTK001 | 2 | pAOX1 |
| pPTK-DAO-1 | 3 | DAO-GH gene |
| pPTK019 | 4 | tAOX1 |
| pYTK072 | 5 | ConRE (assembly connector) |
| pYTK080 | 6 | ZeocinR |
| pPTK020 | 7 | *att8* (Bxbl recognition site) |
| pYTK084 | 8 | KanR-ColE1 |

The DAO-GH expression cassette plasmid was linearized by *Avril* and homologous recombinant integration into the genome of *K. phaffii* was done as known in the art. Recombinant *K. phaffii* clones (in total 10) were investigated for their intracellular DAO activity after cultivation in BMGY/BMMY medium in deep well plates (96/2,000 µL; Eppendorf AG, Germany) as known in the art.

### Bioreactor cultivation of recombinant K. phaffii for the production of DAO-GH

The recombinant *K. phaffii* strain with the highest intracellular DAO-GH activity was used for a fed-batch bioreactor cultivation in Multifors 2 bioreactors (1.4 L vessel volume; Infors HT, Switzerland), as known in the art with slight modifications. The depletion of glycerol was not verified quantitatively, but by an increase in the pO₂ signal. Also, the methanol feed rates were modified. After 21.6 h of batch cultivation on glycerol the methanol feed was started with a rate of 1.8 mL/h for 3 h, followed by feed rates of 3.6 mL/h for 5.2 h, 5.4 mL/h for 2.7 h, 7.2 mL/h for 1.1 h, 5.4 mL/h for 1.8 h, 7.2 mL/h for 5.3 h and 10.1 mL/h for 42.6 h. Regularly, the absence of an accumulation of methanol was qualitatively verified by pausing the methanol feed and observing the pO₂ signal. In case of an immediate increase of the pO₂ signal it was concluded that no methanol was accumulated and the feed rate was either maintained or further increased. Samples of 5 mL were taken regularly during the cultivation and centrifuged (13,000 g, 5 min, 4 °C). The cell pellets were washed with saline and stored at -20 °C until cell disruption. At the end of the bioreactor cultivation the remaining cells were harvested by centrifugation (6,000 g, 15 min, 4 °C), washed with saline and again centrifuged (8,000 g, 15 min, 4 °C). The cell pellet was stored at -20 °C until further use.

### Disruption of K. phaffii cells

*K. phaffii* cells obtained from the deep well plate cultivation were suspended in PIPES buffer (25 mM, pH 7.2) and disrupted in microtiter plates as known in the art.

Cells obtained over the course of the bioreactor cultivation were suspended (30 % (w/v)) in PIPES buffer (25 mM, pH 7.2) and disrupted with the TissueLyser II (Qiagen, Germany) at 30 Hz for 30 min. The supernatant obtained after centrifugation (13,000 g, 5 min, 4 °C) was directly used for the investigation of DAO activity and protein content. For the purification of DAO-GH, a 30 % (w/v) suspension with 264 g *K. phaffii* bio wet mass was prepared and disrupted using the DYNOO-MILL KDL A (Willy A. Bachofen GmbH, Germany) with glass beads (0.75 mm diameter) as known in the art. After centrifugation (10,000 rpm, 45 min, 4 °C), the supernatant was used for the DAO-GH purification.

### Purification of DAO-GH

The DAO-GH was purified by precipitation of nucleic acids using polyethyleneimine, fractionated ammonium sulphate precipitation and hydrophobic interaction chromatography, as known in the art with minor modifications. All centrifugation steps were done at 10,000 rpm, 4 °C for 45 minutes. After polyethyleneimine precipitation of nucleic acids, the fractionated ammonium sulphate precipitation was done by increasing the ammonium sulfate saturation to 25 % to remove foreign proteins and afterwards to 60 % to finally precipitate the DAO-GH. The obtained DAO-GH pellet was dissolved in binding buffer (25 mM sodium phosphate containing 1.3 M (NH₄)₂SO₄) in a final volume of 356 mL and purified by hydrophobic interaction chromatography using the column material Toyopearl Phenyl-650 M (Tosoh Bioscience, Japan) (CV = 350 mL).

### Protein analysis

The protein content of enzyme samples was determined according to Bradford, using BSA as a standard. Samples of the DAO purification procedure were analyzed by sodium dodecyl sulfate (SDS)-polyacrylamide gel electrophoresis (PAGE) on a 10 % separating gel. An amount of 5 µg protein was loaded onto each lane of the SDS-PAGE. A protein molecular mass standard was used (Precision Plus Protein^{™} unstained protein standard 10 - 250 kDa) for molecular mass determination. Coomassie Brilliant Blue R-250 was used to stain the gel.

### DAO activity determination

The DAO-GH activity was determined using the colorimetric DA-67 enzyme assay, as known in the art. The reaction mixture, containing 375 µL histamine solution (2.79 mM; dissolved in 25 mM potassium phosphate buffer; pH 6.8) and 363 µl DA-67 reagent (10-(carboxymethyl-aminocarbonyl)-3,7-bis(dimethylamino) phenothiazine sodium salt; 50 µM; dissolved in 25 mM potassium phosphate buffer; pH 6.8) was incubated at 37 °C for 10 min and stirred at 850 rpm. Subsequently, 12 µL (266 units·mL⁻¹) of horseradish peroxidase (Grade I) (Carl Roth, Germany) were added. The reaction was started by the addition of 25 µL DAO solution and incubated at 37 °C and 850 rpm. The reaction was stopped by the addition of 25 µL sodium diethyldithiocarbamate (30 mM). The absorption was measured at 620 nm. The histamine solution was replaced with buffer (25 mM potassium phosphate buffer; pH 6.8) for reference. Hydrogen peroxide (0.5 - 10 nmol·mL⁻¹) was used for the calibration. The enzyme activity was calculated in nkat, whereby 1 nkat converts 1 nmol substrate·s⁻¹ at 37 °C.

### Investigation of the temperature and pH profile of DAO-GH

The influence of temperature on the enzyme activity of DAO-GH was investigated under standard assay conditions with histamine and tyramine as substrates. The incubation temperature was varied between 5 and 70 °C. The pH-dependency of the DAO-GH activity was investigated under standard assay conditions at 37 °C with histamine and tyramine as substrates. The following buffer systems were used (each at 50 mM): sodium acetate buffer (pH 4.5, 5.0, 5.5, 6.0), MES buffer (pH 5.5, 6.0), potassium phosphate buffer (pH 6.0, 6.5, 6.8), PIPES buffer (pH 6.8, 7.0, 7.5), TRIS buffer (pH 7.5, 8.0, 8.5, 9.0). The respective buffers were used for separate calibrations with hydrogen peroxide (0.5 to 10 nmol/mL).

### Kinetic characterization of DAO-GH

The apparent kinetic parameters of DAO-GH were determined by Michaelis-Menten kinetics with histamine and tyramine as the substrates under standard assay conditions. The histamine concentration was varied between 0.05 and 3.1 mM and the tyramine concentration was varied between 0.005 and 3 mM. Kinetic investigations were done within the initial reaction velocity.

### Example 1:

### Discovery of the new diamine oxidase (DAO-GH) in Glutamicibacter halophytocola

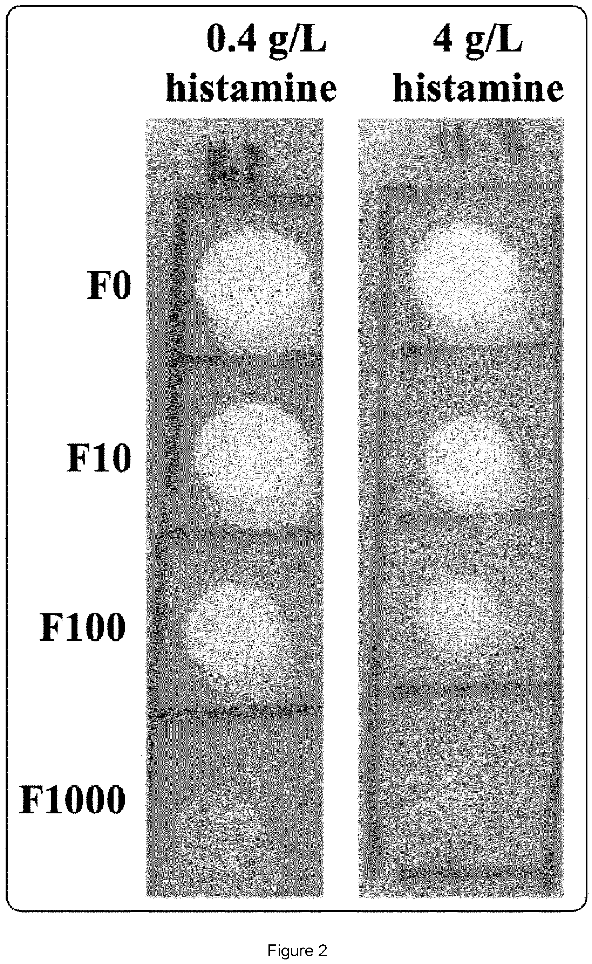

The screening for diamine oxidase producing microorganisms from a soil sample revealed four candidates with different colony morphologies as observed on the agar plate. These candidates were further selected on selective minimal media agar plates with a reduced histamine concentration (0.4 g/L) to screen for microorganisms producing a DAO with a potentially higher affinity to histamine. One candidate seemed to grow better at this reduced histamine concentration in comparison to the higher concentration (Fig. 2) and also showed highest native DAO activity in this screening (14 pkat/mL_{enzyme solution}). This candidate was identified as *Glutamicibacter halophytocola* by 16s rDNA sequencing.

The *G*. *halophytocola* candidate from this work was cultivated in a shake flask in glycerin-supplemented S1 complex media to investigate its native DAO activity. After around 6 hours of cultivation, an optical density (OD₆₀₀) of 0.8 and an intracellular DAO activity of 0.65 nkat/L_{Culture} was determined (no data shown).

The gene coding for a putative "primary amine oxidase" (accession: WP_060702819.1) in *G. halophytocola* was identified using the BLAST program (https://blast.ncbi.nlm.nih.gov) with the amino acid sequences of the DAOs from *Yarrowia lipolytica* (accession: Q6CGT2) and *Arthrobacter crystallopoietes* (accession: WP_074701741.1) as entries. Comparing the amino acid sequences of the DAOs from Y. *lipolytica* and *A. crystallopoietes,* the putative "primary amine oxidase" from *G. halophytocola* showed a percent identity of 39 % (query cover 64 %) and 62 % (query cover 97 %), respectively. The gene coding for the putative primary amine oxidase in *G. halophytocola* was amplified from its genome (99 % percent identity with amino acid sequence of WP_060702819.1) and used for the heterologous recombinant expression in *Escherichia coli* BL21(DE3) and *Bacillus subtilis* SCK6 in preliminary experiments (no data shown). The recombinant expression proved that the putative primary amine oxidase is an active DAO. However, low activity yields of 3 nkat/L_{Culture} (0.01 nkat/mg_{Protein}) in *E*. *coli* and 64 nkat/L_{Culture} (0.23 nkat/mg_{Protein}) in 8. *subtilis* were found.

### Example 2:

### Production of the DAO-GH in Komagataella phaffii

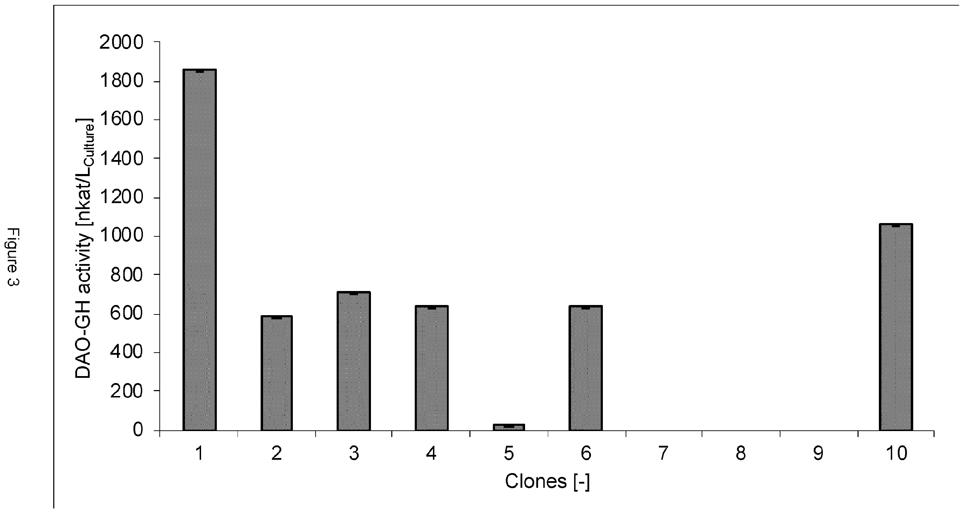

Heterologous DAO-GH production was done in *K. phaffii* using the methanol-inducible AOX1 promoter. Initially, intracellular DAO-GH production in the recombinant *K. phaffii* clones was investigated in deep-well plate cultivations as known in the art (Fig. 3). The clone 1 with the highest DAO-GH activity (1.86 ± 0.018 µkat/L_{culture}) in this deep-well plate screening was used for all further experiments.

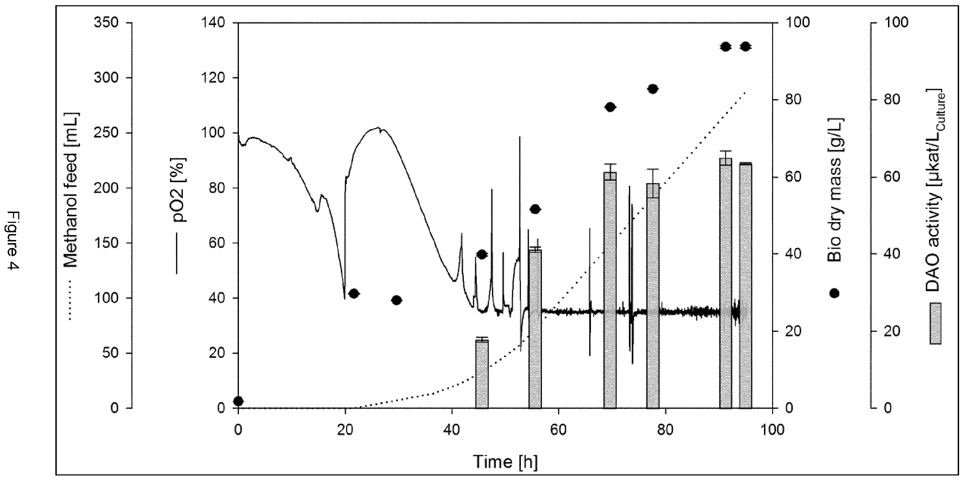

The recombinant *K. phaffii* clone was used for DAO-GH production in a fed-batch bioreactor cultivation. Here, the recombinant *K. phaffii* was first grown on glycerol as carbon source to generate biomass. After glycerol was depleted (indicated by an increase in pO₂), the methanol feed was started, inducing the DAO-GH expression. The methanol feed was step-wise increased and paused or reduced whenever methanol was accumulating ((≤ 1 % (v/v) methanol) (Fig. 4). The maximum volumetric DAO activity of around 65 µkat/L_{culture} (circa 3.2 µkat/gₚᵣₒₜₑᵢₙ) was determined after around 90 hours of cultivation. The bio dry mass at this time point was at around 93 g/L, resulting in a specific DAO activity per gram bio dry mass of 0.7 µkat. The volumetric DAO activity was almost 35-fold higher than the activity observed in the deep-well plate screening.

In comparison, the recombinant production of another DAO, the DAO-1 from *Yarrowia lipolytica,* in *K. phaffii* as described in the art, yielded a volumetric DAO activity of around 230 µkat/L_{culture}. However, the activity of the DAO-1 was investigated under optimal conditions for maximal DAO-1 activity (pH 7.2, 37 °C, 14.52 mM histamine), whereas the activity of the DAO-GH in this study was investigated under conditions that were chosen in prior studies as 'physiologically-relevant conditions' (pH 6.8, 37 °C, 1.35 mM histamine). It has to be considered, that the activity of the DAO-1 from Y. *lipolytica* is about 50 % lower under these conditions (no data shown).

### Example 3:

### Purification of DAO-GH

The DAO-GH produced in the fed-batch bioreactor cultivation of the recombinant *K. phaffii* was purified by polyethyleneimine precipitation of nucleic acids, fractionated ammonium sulfate precipitation and hydrophobic interaction chromatography (HIC). From 264 g of *K. phaffii* bio wet mass, 41 µkat of DAO-GH activity (specific DAO-GH activity of 3.2 µkat/gₚᵣₒₜₑᵢₙ) was obtained after cell disruption by bead mill.

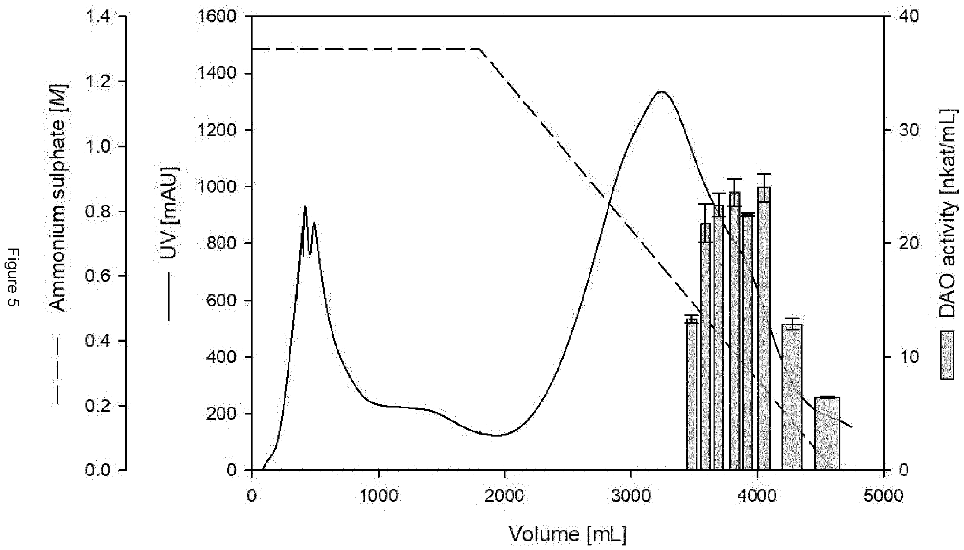

The purification of the DAO-GH by HIC (Fig. 5) yielded a total of around 20 µkat (48 % yield) with a specific DAO-GH activity of 8.8 µkat/g_{Protein} (purification factor of around 3).

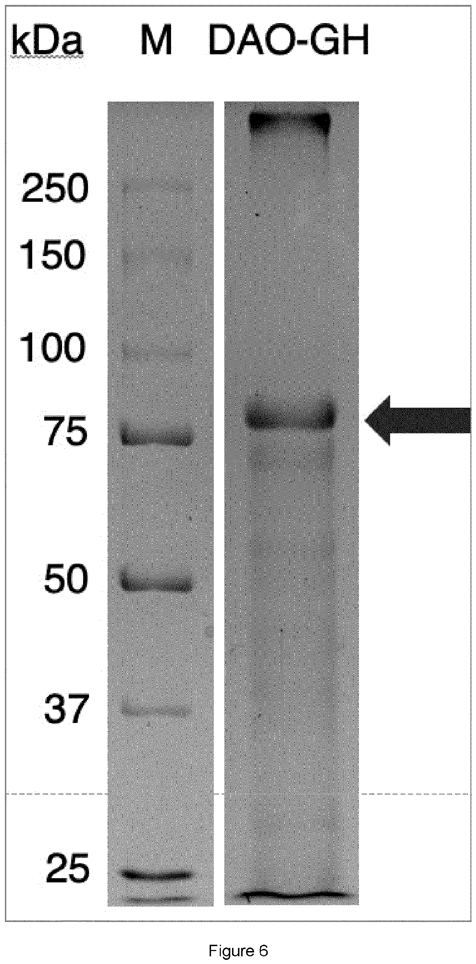

The purified DAO-GH was investigated by SDS-PAGE analysis and showed a distinct band slightly above 75 kDa (Fig. 6). The theoretical molecular weight of the DAO-GH is 72.48 kDa (calculated from the amino acid sequence).

### Example 4:

### Biochemical investigation of DAO-GH

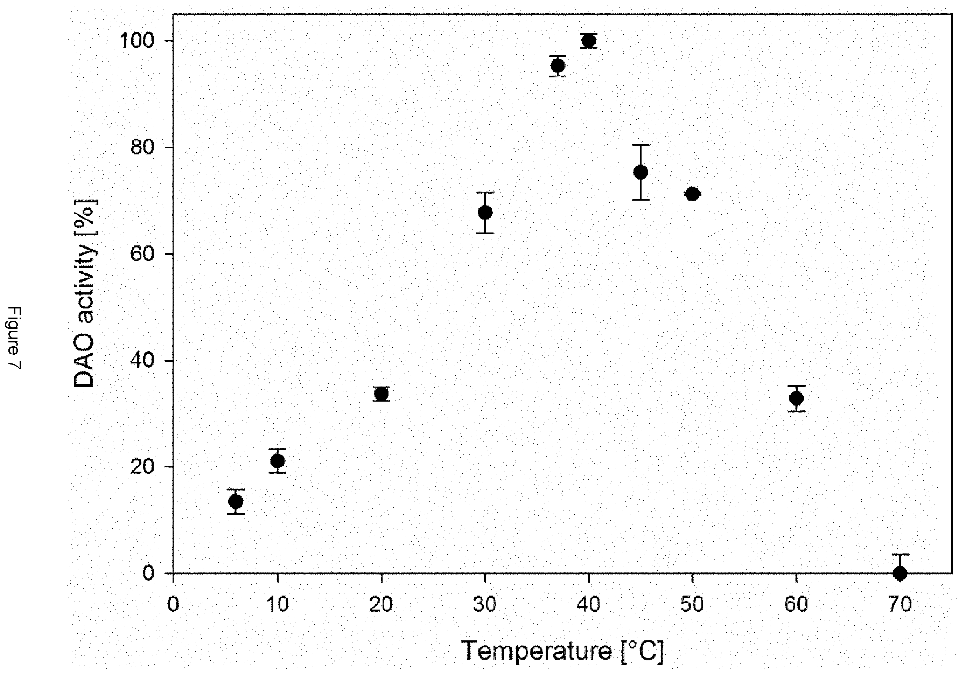

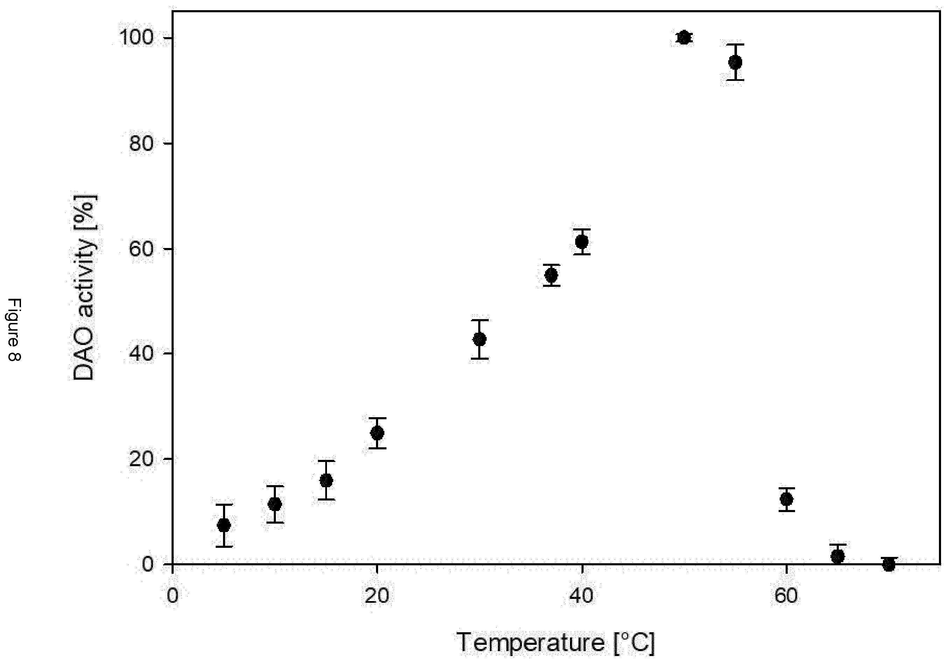

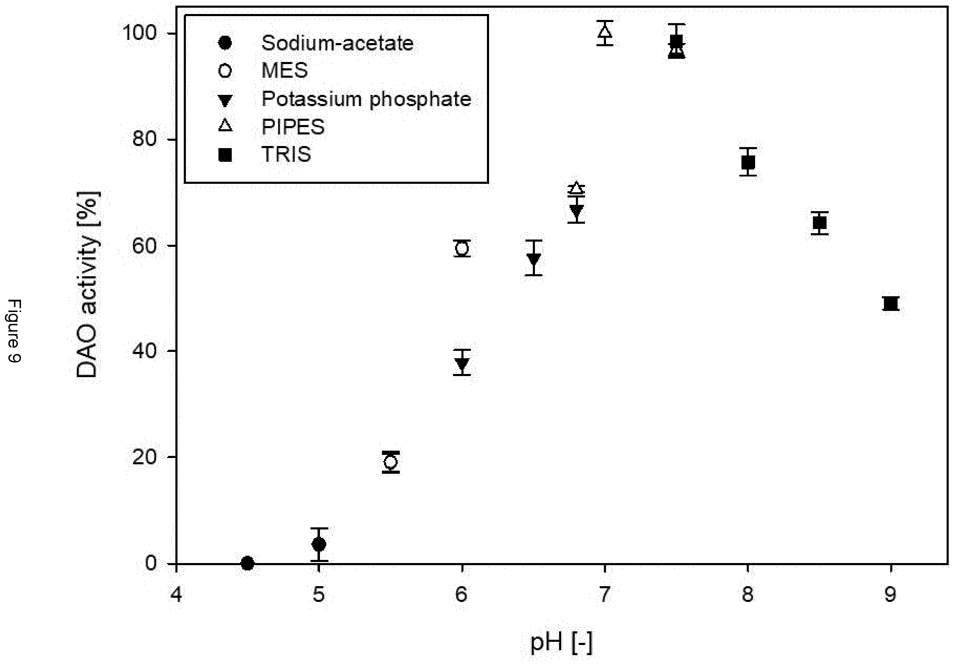

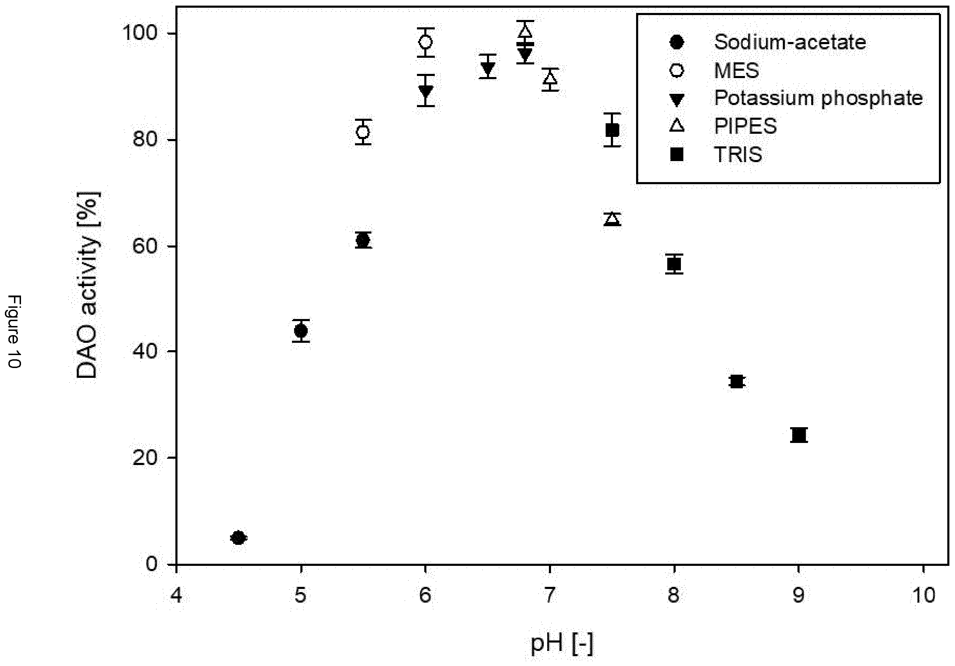

The DAO-GH showed rather broad temperature- and pH-profiles when compared to other DAOs (Figs. 7 to 10). The maximal DAO-GH activity with histamine as substrate was at 40 °C and pH 7.0 in PIPES buffer. For tyramine, the maximal DAO-GH activity was at 50 °C and pH 6.8 in PIPES buffer. Interestingly, the DAO-GH still showed around 14 % of its maximal activity at 5 °C (histamine as substrate). In comparison, a recently published new DAO from Y. *lipolytica* showed around 10 % of its maximal activity at 20 °C. Also, the DAO-GH shows sufficient activity at a neutral to slightly acidic pH-value, which is desirable for the application in fermented foods. Even at a pH of 4.5 and 5.0, the DAO-GH still showed around 5 % and 44 % with tyramine as substrate, respectively. With histamine, the DAO-GH showed around 4 % and 20 % of its maximal activity at pH 5.0 and 5.5, respectively. In contrast, other microbial DAOs like for example the DAO-1 from Y. *lipolytica,* a phenylethylamine oxidase from *Arthrobacter globiformis* or a monoamine oxidase from *Mycobacterium* sp. strain JC1 show no or weak activity in this pH-range.

In conclusion, the DAO-GH is applicable for the histamine reduction in fermented foods, which exhibit an acidic pH due to lactic acid formation and a temperature of up to around 12 °C.

The maximum DAO-GH activity was at pH 7 (histamine) and 6.8 (tyramine) and sufficient activity was present at 37 °C. Hence, the DAO-GH can also be orally administered to degrade dietary biogenic amines like histamine in the human small intestine.

### Example 5:

### Kinetic investigation of DAO-GH

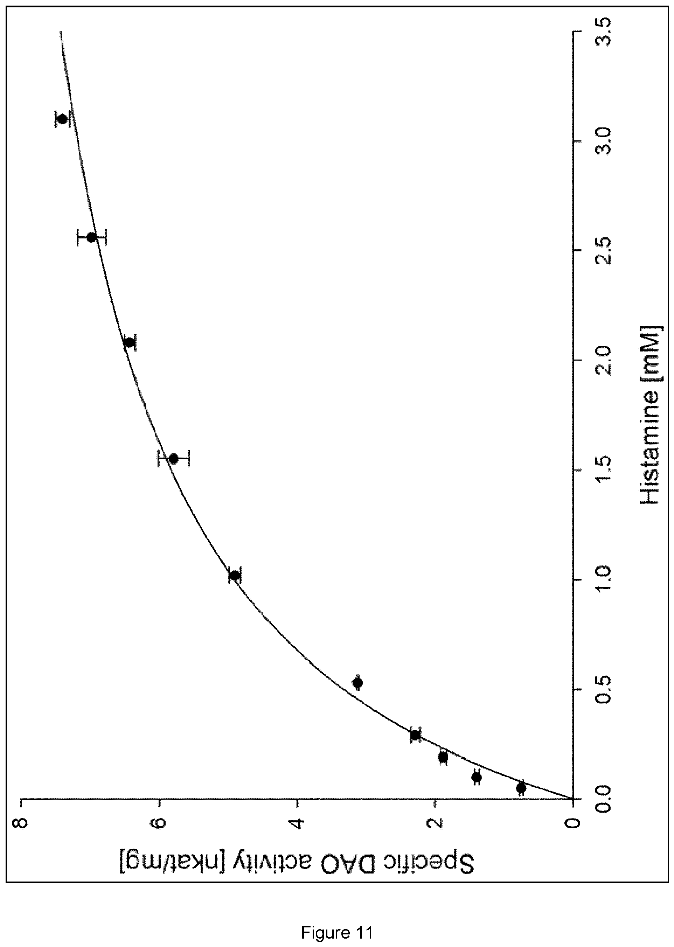

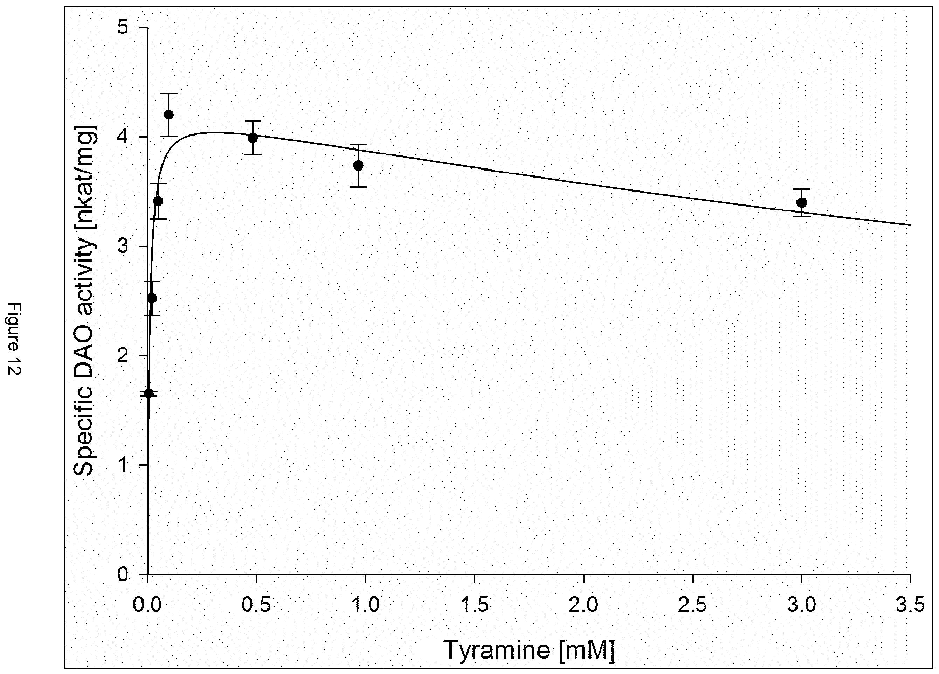

The DAO-GH was investigated regarding its affinity to the substrates histamine (Fig. 11) and tyramine (Fig. 12) (Michaelis-Menten kinetics).

The *K*ₘ of the DAO-GH with histamine as substrate was 0.9 ± 0.01 mM (Vₘₐₓ = 9.3 nkat_{Histamine}/mg_{Protein}; R²=0.986). The DAO-GH was not substrate-inhibited by histamine. In contrast, the DAO-1 from Y. *lipolytica* showed a higher *K*ₘ of 2.3 mM with histamine and was substrate inhibited, which was recognized for histamine concentrations greater than around 10 mM.

Although the DAO-GH showed good affinity towards the substrate histamine, it showed even higher affinity to tyramine with a *K*ₘ of 0.009 ± 0.0002 mM (Vₘₐₓ = 4.3 nkat_{Tyramine}/mg_{Protein}; R²=0.89). In comparison, other oxidases capable of degrading tyramine showed a distinctively lower affinity like for example an amine oxidase from *Aspergillus niger* (*K*ₘ = 0.12 mM) and plant amine oxidases from *Lathyrus cicero* (*K*ₘ = 3.1 mM) and *Pisum sativum* (*K*ₘ = 2.3 mM).

The DAO-GH was substrate inhibited by tyramine (*K*ᵢ = 10.4 mM), which was recognized for tyramine concentrations above approximately 0.5 mM.

Additionally, the DAO-GH activity was determined with the biogenic amines cadaverine and putrescine which are both aliphatic diamines. In concentrations ranging from 0.1 mM to 5 mM, the relative DAO-GH activity (compared to the activity with histamine at 1 mM) was merely between 0.1 to 1.3 % and 2.3 % for cadaverine and putrescine, respectively.

Since the DAO-GH showed highest activity with histamine and tyramine but only weak activity with cadaverine and putrescine, the DAO-GH is selective for the most relevant biogenic amines in the food industry. As stated by the European Food Safety Authority's (EFSA) Panel on Biological Hazards, "histamine and tyramine are considered as the most toxic and food safety relevant" biogenic amines (EFSA, 2011). Tyramine exerts effects of vasoconstriction in the human body causing symptoms like hypertension, headache, perspiration, vomiting and pupil dilation (EFSA, 2011). Since these symptoms are also experienced in the case of histamine intolerance, the differentiation to a tyramine intolerance is inconclusive.

The present invention relates to the following amino acid and nucleotide sequences:
**SEQ ID NO: 1**
   **Amino acid sequence of DAO-GH from *Glutamicibacter halophytocola*** 
MEHLHPTTALETAHPLEQITSEEILRTRKILADAGLVEQTTRFAYLGLLDPPKDLLYAD
AGTEIPRKIRVMLYDPTIPRSLDITICLASAEIESQREIEAATEGQVPVLLEEFDTVEE
ILANDEGWIKALASRGLSTSQVRVAPLSAGVFDYENEEGKRLLRGLGFVQNSPEDHAWA
HPIDRLVAFVDLENRCVDRLIDDGPVPVPDINGNYTDPQVHGELRDDLKAIEITQPDGA
SFTVDGNHLSWLGWDLRVGFDSREGLVLHQIHHTQDGTRRPLIHRASISEMVVPYGDPS
PYRSWQNYFDTGEYLVGRDANSLKLGCDCLGEIHYMSPMVADDFGNPRVIDNGICIHEE
DAGIGWKHTDEWAGSNEVRRNRRLVVSFFTTVGNYDYGFYWYLYLDGTIEFEAKATGIV
FTAALPHKGYEYASEIAPGLAAPFHQHLFGARLDMMIDGHANAVDELEVVRLPKSEGNP
HGNAFTQSRLRLGTEQQAVRDANAAAGRVWQVSNPDSLNHVGEPVGYTLYPQNNPTLAM
ADDSSIAARAAFTRHDLWVTRFAEGELYAAGDFVNRNPGGAGLPAFVEADRDIDGQDIV
LWHSFGLTHFPRPEDWPIMPVDTVGFTLKPHGFFNENPMLNIPASTSSHCSMQAPETEG
HCGA
**SEQ ID NO: 2**
   **Gene encoding DAO-GH** 
atggaacaccttcacccaacgacagcgttggagaccgcgcacccactggaacagatcac
cagtgaagagatcctgcgtactcgcaagatccttgccgacgccgggctggttgagcaga
ccacccgtttcgcctacctgggcttgctggatccacccaaggacctgctctatgccgat
gctggcacagagattccgcgcaagattcgtgtgatgctctacgatccgaccatcccccg
ctccctggacatcacgatctgcctggccagcgccgagatagaatcacagcgcgaaatcg
aagcagcgacagagggacaagttccggtgctgcttgaagaattcgacaccgtcgaagaa
atcctggccaacgacgaaggatggatcaaggccttggccagccgcggactgagcacatc
ccaggttcgagtcgcaccgctgtcagcaggtgtcttcgactacgagaacgaagaaggaa
aacgcctattgcgcggactgggcttcgtgcagaattcccctgaggaccacgcttgggcc
caccccatcgatcgcttggtggcctttgtcgacctcgaaaaccgttgcgttgaccggct
catcgatgatggccctgttccagttcccgacatcaacggaaactacactgatccacaag
ttcatggtgagctgcgcgatgatctcaaggccatcgaaatcacccagccagatggagcc
agcttcaccgtggatggcaaccacctctcgtggctcggctgggatctgcgcgtcggctt
tgactcacgcgaaggactcgtactccaccagatccaccacacgcaagacggcacgcgtc
gcccgctgatccaccgcgcatccatctccgaaatggtagtgccctacggcgatccgtcc
 
ccttaccggagctggcagaactatttcgacaccggagaatatttggtgggacgcgacgc
gaactcactgaagctcggctgcgactgcctcggagaaatccactacatgtctcccatgg
ttgccgacgattttggcaatcctcgcgtcatcgacaacggcatctgcattcacgaggaa
gacgccggaatcggatggaagcacaccgatgagtgggccggatccaacgaagtgcgccg
caaccggcgcctggtggtttccttcttcaccaccgtgggcaattatgactacggtttct
actggtacctgtacctcgacggcaccatcgagtttgaagccaaggccacgggcatcgtc
ttcaccgcggcgttgccgcacaagggttacgagtacgcctcagaaatcgcgccgggctt
ggctgcgccattccaccagcatctctttggcgcacgccttgacatgatgatcgacgggc
acgccaacgctgttgacgagctagaagtagtccggttaccgaagtccgaaggcaatcca
cacggcaacgccttcacccaaagccggctgcgtttgggcaccgagcagcaggcggtacg
cgacgccaatgcagcggcgggacgcgtatggcaggtgagcaaccccgattccctgaatc
acgtgggcgagccggtgggctacacgttgtacccgcagaacaatccgacgctggctatg
gctgacgactcgtccatcgctgcccgtgccgcctttacccgacacgacctctgggtcac
gcgctttgccgaaggcgagctctatgcagccggcgacttcgtcaaccgcaatccaggcg
gggcggggcttcctgccttcgtcgaggccgaccgggatatcgacggacaagatatcgtg
ttgtggcactcctttggcctgacccacttccctcgccccgaggactggcccatcatgcc
agtggataccgtagggttcacgctgaagccacacggtttcttcaatgaaaacccgatgc
tcaatatccctgcctcgacaagctcgcactgttccatgcaagctcctgaaaccgaagga
cactgcggagcctag
**SEQ ID NO: 3**
   **Gene encoding DAO-GH (codon-optimized for *Komagataella phaffii*)** 
atggaacacttgcacccaactactgctttggaaactgctcaccctttggagcagattac
ctccgaagagatcctgagaaccagaaagattttggctgacgccggtttggttgagcaga
ctactagatttgcctacttgggtttgctggacccaccaaaggatttgttgtacgctgat
gctggtactgagatcccaagaaagatcagagtcatgctgtacgacccaactatcccaag
atccttggacattaccatctgtttggcttccgctgagattgagtcccagagagaaattg
aagctgctaccgaaggtcaggtcccagttttgttggaagagttcgacaccgtggaagag
atcttggctaacgacgaaggttggatcaaggctttggcttctagaggtttgtccacctc
tcaggttagagttgctccattgtctgctggtgttttcgactacgaaaacgaggaaggta
agagactgttgagaggtttgggtttcgttcagaactctccagaagatcacgcttgggct
cacccaattgatagattggttgccttcgtcgacttggagaacagatgtgtcgacagatt
gattgacgacggtccagttccagttcctgacatcaacggtaactacactgacccacaag
 
ttcacggtgagttgagagatgacttgaaggctatcgagatcactcaaccagacggtgct
tctttcactgttgacggtaaccacttgtcttggcttggttgggatttgagagttggttt
cgactccagagaaggtttggtcttgcaccagattcaccacactcaagacggtactagaa
ggccattgattcacagagcttccatttccgagatggtcgttccatacggtgatccatct
ccatacagatcctggcagaactacttcgacactggtgagtacttggttggtagagatgc
caactccttgaagttgggttgtgactgtttgggtgagatccactacatgtccccaatgg
ttgctgatgacttcggtaacccaagagtcatcgacaacggtatctgtatccacgaagag
gacgctggtattggttggaagcacactgatgaatgggctggttctaacgaggtcagaag
aaacagaagattggtcgtttccttcttcaccaccgttggtaactacgactacggtttct
actggtacttgtacctggacggaactatcgagttcgaggctaaggctactggtatcgtt
ttcactgctgctttgccacacaagggttacgaatacgcttctgagattgctccaggttt
ggctgctccatttcaccaacatttgttcggtgccagactggacatgatgattgatggtc
atgctaacgccgttgacgagttggaggttgttagattgccaaagtctgagggtaaccca
cacggtaacgctttcactcagtccagattgagattgggtactgagcagcaggctgtcag
agatgctaatgctgctgctggtagagtttggcaggtttctaacccagactctttgaacc
acgttggtgagccagttggttacactctgtacccacaaaacaaccctactttggctatg
gctgacgactcttccattgctgctagagctgctttcactagacacgatttgtgggtgac
tagattcgctgagggtgagttgtatgctgccggtgatttcgttaacagaaacccaggtg
gtgctggtttgccagcttttgttgaagctgacagagacattgacggtcaggacatcgtt
ttgtggcattcctttggtctgactcacttcccaagacctgaggactggccaattatgcc
agttgacactgttggtttcaccttgaagccacacggtttcttcaacgagaacccaatgt
tgaatatcccagcctccacttcttcccactgttctatgcaagctccagaaactgaaggt
cactgtggtgcttaa
**SEQ ID NO: 4**
   **Primer sequence**
   atggaacaccttcacccaacga
**SEQ ID NO: 5**
   **Primer sequence**
   ctaggctccgcagtgtccttcggtttcagg

## Claims

1. A functional food or dietary supplement, comprising an enzyme having diamine oxidase activity, wherein said enzyme comprises
(i) the amino acid sequence of SEQ ID NO: 1; or
(ii) an amino acid sequence having at least 70% sequence identity to SEQ ID NO: 1 and having diamine oxidase activity.

2. Use of an enzyme having diamine oxidase activity
(i) in the production of a biogenic amine-depleted product, or
(ii) as a biosensor for the analysis of one or more biogenic amine(s), wherein said enzyme comprises
(α) the amino acid sequence of SEQ ID NO: 1; or
(β) an amino acid sequence having at least 70% sequence identity to SEQ ID NO: 1 and having diamine oxidase activity.

3. A method for the production of a biogenic amine-depleted product, comprising the step of contacting (i) a biogenic amine-containing product, and/or (ii) a biogenic amine-containing intermediate product of said product, with an enzyme having diamine oxidase activity under conditions and for a duration of time suitable to degrade a biogenic amine present in said product and/or in said intermediate product of said product, wherein said enzyme comprises
(i) the amino acid sequence of SEQ ID NO: 1; or
(ii) an amino acid sequence having at least 70% sequence identity to SEQ ID NO: 1 and having diamine oxidase activity.

4. An enzyme having diamine oxidase activity for use in medicine, wherein said enzyme comprises
(i) the amino acid sequence of SEQ ID NO: 1; or
(ii) an amino acid sequence having at least 70% sequence identity to SEQ ID NO: 1 and having diamine oxidase activity.

5. The functional food or dietary supplement according to claim 1, the use according to claim 2, the method according to claim 3, or the enzyme for use according to claim 4, wherein the enzyme consists of
(i) the amino acid sequence of SEQ ID NO: 1; or
(ii) the amino acid sequence having at least 70% sequence identity to SEQ ID NO: 1 and having diamine oxidase activity.

6. The functional food or dietary supplement according to claim 1 or claim 5, the use according to claim 2 or claim 5, the method according to claim 3 or claim 5, or the enzyme for use according to claim 4 or claim 5, wherein the amino acid sequence in (ii) has at least 72%, at least 74%, at least 76%, at least 78%, at least 80%, at least 82%, at least 84%, at least 86%, at least 88%, at least 90%, at least 91%, at least 92%, at least 93%, at least 94%, at least 95%, at least 95.5%, at least 96%, at least 96.5%, at least 97%, at least 97.5%, at least 98%, at least 98.2%, at least 98.4%, at least 98.6%, at least 98.8%, at least 99%, at least 99.1%, at least 99.2%, at least 99.3%, at least 99.4%, at least 99.5%, at least 99.55%, at least 99.6%, at least 99.65%, at least 99.7%, at least 99.75%, at least 99.8%, at least 99.85%, at least 99.9%, at least 99.91%, at least 99.92%, at least 99.93%, at least 99.94%, at least 99.95%, at least 99.96%, at least 99.97%, at least 99.98%, or at least 99.9% sequence identity to SEQ ID NO: 1.

7. The functional food or dietary supplement according to any one of claims 1, 5, and 6, the use according to any one of claims 2, 5, and 6, the method according to any one of claims 3, 5, and 6, or the enzyme for use according to any one of claims 4 to 6, wherein the enzyme consists of the amino acid sequence of SEQ ID NO: 1.

8. The use according to any one of claims 2, and 5 to 7, or the method according to any one of claims 3, and 5 to 7, wherein the product is selected from the group consisting of foodstuffs and feedstuffs.

9. The use according to claim 8, or the method according to claim 8, wherein the product is a foodstuff, selected from the group consisting of fermented foodstuffs, cheeses, sauerkraut, sausages, wine, chocolate, yeast extracts, fish, fish products, raw meats, vegetables, dairy products, and fresh milk.

10. The enzyme for use according to any one of claims 4 to 7 for use in a method of preventing or treating a condition or disease that is associated with increased levels of biogenic amines in a subject.

11. The enzyme for use according to claim 10, wherein the increased levels of biogenic amines are due to the ingestion of said biogenic amines.

12. The enzyme for use according to claim 10 or claim 11, wherein the condition or disease that is associated with increased levels of biogenic amines is selected from the group consisting of allergies, acute and chronic allergic diseases, allergic reactions, allergy-like reactions, itching (pruritus), diarrhoea, redness (erubescence), vomiting (emesis), acute and chronic biogenic amine poisoning, hypotonia, difficulty of breathing, biogenic amine intolerance, anaphylaxis, anaphylactic shock, acute and chronic urticaria, asthma, hay fever, allergic rhinitis, allergic conjunctivitis, headache, migraine, atopic dermatitis, mastocytosis, mast cell activation syndrome (MCAS), pre-eclampsia, hyperemesis gravidarum, pre-term labour, peptic ulcers, acid reflux, sepsis, fibromyalgia, chronic fatigue syndrome, and spondylitis.

13. The functional food or dietary supplement according to any one of claims 1, and 5 to 7, the use according to any one of claims 2, and 5 to 9, the method according to any one of claims 3, and 5 to 9, or the enzyme for use according to any one of claims 4 to 7, and 10 to 12, wherein the biogenic amine is selected from the group consisting of histamine, tyramine, putrescine, and cadaverine.

14. The functional food or dietary supplement according to claim 13, the use according to claim 13, the method according to claim 13, or the enzyme for use according to claim 13, wherein the biogenic amine is selected from the group consisting of histamine and tyramine.

15. The functional food or dietary supplement according to claim 14, the use according to claim 14, the method according to claim 14, or the enzyme for use according to claim 14, wherein the biogenic amine is histamine.
